# EUROPEAN PATENT APPLICATION

(11) **EP 2 668 853 A1**
(43) Date of publication of application: **04.12.2013**
(21) Application number: 13169711.2
(22) Date of filing: 29.05.2013
(51) Int. Cl.: A23L 1/30, A61K 36/15, A61P 11/00

(54) **Method for preparing syrup from fir tree cones and resulting composition.**

(30) Priority: 29.05.2012 ES 201230815
(71) Applicant: Duro Vidal, Josep, 500 Andorra la Vella (AD)
(72) Inventor: Duro Vidal, Josep, 500 Andorra la Vella (AD)
(74) Representative: Carvajal y Urquijo, Isabel

(57) **Abstract**

The object of the present invention consists of a composition and method which allow fir tree syrup to be produced for therapeutic and dietary purposes. The elaboration process begins with a first stage of harvesting the fir cones, which are arranged in a ratio of between 5 and 12 kg of fir cones (depending on their maturity) to every 9 kg of sugar, in a lidded container.

The mixture formed is left to macerate for approximately 8 months at a controlled temperature of between 10 and 18 °C.

Once the 8 month period has elapsed, the result is filtered in order to eliminate solid residue and the filtered material is left to rest for one month, with the aim of obtaining an ideal texture.

## Description

### Object of the invention

The object of the present invention consists in a composition and method that make it possible to produce fir tree syrup for therapeutic and dietary purposes.

### Background of the invention

There are many types of Fir Trees, all of them being medicinal plants. They belong to the coniferous family, which enjoy a good reputation in terms of the healing and vivifying properties they possess.

With the leaves, pines and new shoots of the fir tree, various kinds of tea may be prepared, which have a cleansing effect in the blood. In addition, it serves to strengthen the respiratory system, greatly helping asthma sufferers, who are recommended to take long walks in pine forests and if possible, to keep cut pine branches in a vase in their bedrooms, since their aroma purifies and energizes the environment.

In order to prepare the tea, the leaves, pines and shoots may be used, as mentioned, 20 grams of which are to be placed in a recipient before 1 litre of boiling water is added, leaving it to rest for half an hour. Later, it will be filtered in order to be able to drink one cup every 6 hours, which is sweetened with honey from bees or treacle.

Fresh juice may also be obtained from the shoots, young branches and pine cones of the fir tree, either by manually pressing them or using a household juice extraction machine. Half a teaspoonful may be taken every 2 hours if necessary, in the case of rebellious diseases. Health will be restored in a short time, in addition to looking after it with a healthy and complete diet. The juice obtained may also be rubbed on painful areas caused by the abovementioned problems, for example in the case of lower back pain, bone pains or knocks. Using this juice also prepares you against bouts of flu or disease of the respiratory system, since it strengthens the body's defences against said illnesses when they strike.

Nevertheless, these infusions do not contain all the nutrients required for them to be applied within the diet industry and for therapeutic purposes.

However, the invention described herein reveals a method for preparing syrup made from fir trees, with characteristic properties and a fundamental composition to be used both as a therapeutic product and as a dietary product.

### Description of the invention

The syrup, object of the present invention, is effective against sore throats, since the syrup has many chest decongestant qualities, which alongside propolis, enhance its catarrh prevention properties.

The syrup, object of the present invention, is useful as a natural condiment for chesses, yogurt and as a sugar substitute in the manufacture of ice-creams, cakes, sweets, etc. Mixing it with vinegar makes it possible to produce balsamic and cocktail vinegars.

The elaboration process is based on a first stage of harvesting the fir tree pine cones, of which there will be between 5 and 12 kg of pine cones (depending on how mature they are) for every 9 kg of sugar in a lidded container.

The resulting mixture is left to macerate for approximately 8 months, at a controlled temperature of between 10 and 18 °C, so that they ferment completely, the mixture being stirred at least monthly throughout this period, in order to ensure a homogenous fermentation.

Once the 8 month interval has passed, the result is filtered to eliminate solid residue and the filtered substance is left to rest for one month, in order to obtain an ideal texture. Should you wish to accelerate the method for obtaining the ideal texture, the filtered material may be placed in a larger container and left to boil for approximately 10 to 15 minutes.

Results of an analysis of the syrup composition, object of the present invention, reveal a glucose content of at least 63.30%, a water content of at least 35.36 % and the remaining content consists of essential oils and traces of resin (alpha pyrenes).

This syrup composition is ideal, in order to use the obtained syrup with the aim of relieving symptoms such as coughs, catarrh and flu and it may also be used for dietary purposes as a condiment for cheese, yogurt, etc. and as a sugar substitute in the manufacture of ice-cream, cakes, sweets, etc.

## Claims

1. Process for preparing Fir Tree syrup, **characterized in that** it entails the following operational stages:
- the fir cones are placed in a closed container, with between 5 to 12 kg of Fir to every 9 kg of sugar.
- the mixture formed in the previous stage is left to macerate for approximately 8 months, at a controlled temperature of between 10 and 18 °C, the mixture being stirred at least once a month;
- once the 8 month period has elapsed, the result is filtered in order to eliminate solid residue and the filtered material is left to rest for one month or placed in a larger container and left to boil for approximately 10 to 15 minutes in order to accelerate the process.

2. Fir tree syrup obtained by the process described in claim 1, **characterized in that** it is formed by fermenting a ratio of fir cones and sugar, composed of at least 63.30% sugar, at least 35.36/% water and the remainder consists of essential oils and traces of resin (Alpha Pyrenees).
